# EUROPEAN PATENT SPECIFICATION

(11) **EP 1 521 967 B1**
(45) Date of publication and mention of the grant of the patent: **06.05.2009**
(21) Application number: 03740791.3
(22) Date of filing: 11.07.2003
(51) Int. Cl.: G01N 33/68

(54) **PLASMA UROTENSIN IN HUMAN HEART FAILURE**
PLASMA UROTENSIN UND MENSCHLICHE HERZERKRANKUNG
UROTENSINE PLASMIQUE DANS UNE INSUFFISANCE CARDIAQUE HUMAINE

(30) Priority: 11.07.2002 GB 0216191; 16.07.2002 GB 0216500; 17.07.2002 GB 0216505
(43) Date of publication of application: 13.04.2005
(73) Proprietor: Inverness Medical Switzerland GmbH, 6300 Zug (CH)
(72) Inventor: NG, Leong, c/o University of Leicester, Leicester LE1 7LX (GB)
(74) Representative: Lee, Nicholas John
(86) International application number: PCT/GB2003/003011
(87) International publication number: WO 2004/008140

(56) References cited:
- EP-A- 1 241 479
- WO-A-00/45176
- DOUGLAS S.A. ET AL.: "Congestive heart failure and expression of myocardial urotensin II." LANCET, vol. 359, 8 June 2002 (2002-06-08), pages 1990-1997, XP004366039
- ZOU Y. ET AL.: "Urotensin II induces hypertrophic responses in cultured cardiomyocytes from neonatal rats." FEBS LETT., vol. 508, 2001, pages 57-60, XP004322277 cited in the application
- HUNT P.J. ET AL.: "Immunoreactive amino-terminal pro-brain natriuretic peptide (NT-PROBNP): a new marker of cardiac impairment." CLIN. ENDOCRIN., vol. 47, 1997, pages 287-296, XP000913471
- DAGGUBATI S. ET AL.: "Adrenomedulin, endothelin, neuropeptide Y, atrial, brain, and C-natriuretic prohormone peptides compared as early heart failure indicators." CARDIOVASC. RES., vol. 36, 1997, pages 246-255, XP000913576
- NG L.L. ET AL.: "Plasma urotensin in human systolic heart failure." CIRCULATION, vol. 106, 3 December 2002 (2002-12-03), pages 2877-2880, XP002263937
- DSCHIETZIG T. ET AL: 'Plasma levels and cardiovascular gene expression of urotensin-II in human heart failure' REGULATORY PEPTIDES vol. 110, 2002, pages 33 - 38
- RITZEL R.A. ET AL: 'Induction of b-cell rest by a Kir6.2/SUR1-selective KATP-channel opener preserves b-cell insulin stores and insulin secretion in human islets cultured at high (11mM) glucose' J. CLIN. ENDOCRINOL. METAB. vol. 89, no. 2, 2004, pages 795 - 805

## Description

The present invention relates to methods for the diagnosis of heart failure in mammalian subjects, such as humans. In particular, it relates to such methods in which the level of urotensin in a sample of bodily fluid is measured.

Heart failure (left ventricular systolic dysfunction) is a leading cause of mortality and morbidity. Its pathophysiology involves activation of many neurohormonal systems, including the catecholamine, renin-angiotensin, endothelin, atrial and brain natriuretic peptide systems. Some of these systems are activated in an adaptive fashion, others are maladaptive.

Recent work has revealed the existence of a novel cardiovascular peptide called urotensin II (UTN) with homology to the hormone of teleosts (Ames et al., Nature 1999; 16 : 282-286). This cyclic undecapeptide is the ligand for an orphan G-protein receptor (GPR14) and both peptide and receptors are distributed within the myocardium, endothelium, vascular myocytes and nervous system (Ames et al., Nature 1999; 16 : 282-286). Although a potent vasoconstrictor for certain vascular beds in rats and monkeys (Ames et al., Nature 1999; 16 : 282-286; Douglas et al. J Cardiovasc Pharmacol 2000; 36 : S163-6), it may be vasodilator in the mesenteric resistance vessels (Bottrill et al. Br J Pharm 2000; 130 : 1865-1870). There are important species differences in the reactivity of different vessels to UTN. For example, although monkey vessels are potently vasoconstricted by UTN, human pulmonary vasculature and mesenteric resistance vessels are vasodilated with high potency by UTN (Stirrat et al. Am J Physiol 2001; 280 : H925-928) and human subcutaneous resistance vessels show no reactivity to UTN (Hillier et al. Circulation 2001; 103 : 1378-1381). Chronic stimulation of vascular smooth muscle by UTN leads to hypertrophic responses (Watanabe et al. J Hypertens 2001 ; 19 : 2191-2196). Direct effects on the myocardium were suggested in monkeys with myocardial depression (Ames et al., Nature 1999; 16 : 282-286) probably resulting from coronary vasoconstriction, although in human heart muscle, a positive inotropic effect was demonstrated (Russell et al. Br J Pharm 2001; 132: 5-9). In addition, hypertrophic effects including increased collagen deposition have been described, suggesting a possible role for UTN in ventricular remodeling of heart failure (Zou et al. FEBS Lett 2001; 508 : 57-60; Tzanidis et al. Eur Heart J 2000: 21 : 72). UTN led to increased expression of the atrial and brain natriuretic peptides in cardiomyocytes (Zou et al. FEBS Lett 2001; 508 : 57-60), a change expected in heart failure with reversion to a more primitive phenotype. Recent work by Douglas *et al* (Douglas et al. Lancet 2002; 359 : 1990-1997) documented increased expression of both UTN and its receptor in the myocardium of patients with heart failure, even in early stages of the disease. UTN was present in cardiomyocytes, vascular smooth muscle cells, endothelium and inflammatory cells within the heart. With regard to a putative role for urotensin in heart failure, the initial positive inotropic and vasodilator effect in humans may be compensatory, before remodeling and increased fibrosis leads to an increasingly maladaptive response.

In a first aspect, the present invention provides a method for the diagnosis of heart failure in a mammalian subject whereby the level of urotensin in a sample of bodily fluid is measured and compared to reference levels typically found in the said bodily fluid of a subject which are indicative of the absence of heart failure, whereby an elevated level of urotensin is indicative of heart failure.

The level of at least a further marker indicative of heart failure, such as N-terminal pro-Brain Natiuretic Peptide (NT-proBNP) or Brain Natiuretic Peptide (BNP) may be additionally measured and compared to reference levels typically found in the bodily fluid of a mammalian subject which are indicative of the absence of heart failure, whereby an elevated level of the at least a further marker is indicative of heart failure.

The level of urotensin or the further marker may be determined by use of an immunoassay, and the bodily fluid may be plasma or interstitial fluid.

In one embodiment, the method of the present invention is for the diagnosis of heart failure in a human subject.

In a second aspect, the present invention provides a method of tracking and staging of the degree of severity of heart failure in a human subject whereby one measurement or more than one measurement over a period of time of the levels of urotensin in a sample of bodily fluid and at least a further marker indicative of heart failure is made and compared to a standard model.

The standard model may be a standard model of levels of analyte versus disease status, and the staging may be determined according to the New York Heart Association (NYHA) classification. This is a four-stage classification where:
Class 1. Patients exhibit symptoms only at exertion levels
Class 2. Patients exhibit symptoms with ordinary exertion.
Class 3. Patients exhibit symptoms with minimal exertion.
Class 4 Patients exhibit symptoms at rest.

The present inventor investigated the plasma levels of UTN in human heart failure (left ventricular systolic dysfunction) and compared them to the more established plasma elevation of N-terminal pro-brain natriuretic peptide (N-terminal proBNP). Plasma was obtained from 126 patients with heart failure and 220 age and gender matched normal controls. N-terminal proBNP and UTN were measured by immunoluminometric assays. Both N-terminal proBNP and UTN were elevated in the plasma of heart failure patients and were significantly correlated (rₛ = 0.35, P<0.001). In contrast to N-terminal proBNP, there was no relation of plasma UTN with NYHA class. Although plasma N-terminal proBNP showed a positive relationship with age and female gender, there was no such age dependent change in plasma UTN and normal females had lower levels compared to males. Receiver operating characteristic curves for the diagnosis of heart failure had areas of 0.90 and 0.86 for N-terminal proBNP and UTN respectively (P<0.001 for both). Plasma UTN is elevated in human heart failure, suggesting a role for this peptide in the pathophysiology of the disease. Plasma UTN can be a useful alternative to N-terminal proBNP in the diagnosis of heart failure as its levels in heart failure are elevated irrespective of age, gender or NYHA class.

In the present invention, the measured level of urotensin (and, where measured, the further marker(s) indicative of heart failure) is compared with reference levels typically found in the said bodily fluid of a subject which are indicative of the absence of heart failure. These reference levels may be determined from population studies of subjects free from heart failure. Such subjects may be matched for age and/or gender.

As mentioned earlier, the further marker may be N-terminal proBNP. Alternatively or additionally, the level of BNP may be measured. The release of stored proBNP (the intact precursor to the two circulating forms, BNP (the active peptide) and N-terminal BNP (NTproBNP the inactive peptide)) from cardiac myocytes in the left ventricle and increased production of BNP is triggered by myocardial stretch, myocardial tension, and myocardial injury.

The bodily fluid in which the level of urotensin is measured may be plasma or interstitial fluid, although other sources of bodily fluid, namely whole blood, serum, or urine may be used.

The markers measured in the present invention may be detected using an immunoassay. In one embodiment, an immunoassay is performed by contacting a sample from a subject to be tested with an appropriate antibody under conditions such that immunospecific binding can occur if the marker is present, and detecting or measuring the amount of any immunospecific binding by the antibody. Any suitable immunoassay can be used, including, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

For example, a marker can be detected in a fluid sample by means of a two-step sandwich assay. In the first step, a capture reagent (e.g., an anti-marker antibody) is used to capture the marker. The capture reagent can optionally be immobilised on a solid phase. In the second step, a directly or indirectly labelled detection reagent is used to detect the captured marker. In one embodiment, the detection reagent is an antibody. In another embodiment, the detection reagent is a lectin.

An immunoassay, kit or device for detection of urotensin may incorporate the method of the first aspect of the present invention. Such an immunoassay kit or device may comprise an antibody which binds specifically to urotensin and optionally an antibody which binds the further marker of heart failure. In addition, such an immunoassay may optionally comprise one or more of the following: (1) instructions for using the immunoassay, kit or device for diagnosis of heart failure; (2) a labelled binding partner to the antibody; (3) a solid phase (such as a reagent strip) upon which the or each antibody is immobilised; and (4) a label or insert indicating regulatory approval for diagnostic, prognostic or therapeutic use or any combination thereof. If no labelled binding partner to the or each antibody is provided, the or each antibody itself can be labelled with a detectable marker, e.g., a chemiluminescent, enzymatic, fluorescent, or radioactive moiety.

The term "antibody" as used herein refers to immunoglobulin molecules and immunologically active portions of immunoglobulin molecules, i.e., molecules that contain an antigen binding site that specifically binds an antigen. The immunoglobulin molecules useful in the invention can be of any class (e.g., IgG, IgE, IgM, IgD and IgA ) or subclass of immunoglobulin molecule. Antibodies includes, but are not limited to, polyclonal, monoclonal, bispecific, humanised and chimeric antibodies, single chain antibodies, Fab fragments and F(ab') fragments, fragments produced by a Fab expression library, anti-idiotypic (anti-Id) antibodies, and epitope-binding fragments of any of the above.

The invention provides a method for the diagnosis of heart failure in a mammalian subject whereby the levels of at least a first marker indicative of heart failure and/or urotensin in a sample of bodily fluid is measured and compared to the reference levels typically found in the said bodily fluid of a mammalian subject which are indicative of the absence of heart failure, whereby an elevated level of the at least a first marker and/or urotensin is indicative of heart failure. The invention also provides a method of tracking and staging of the degree of severity of heart failure in a human subject whereby one measurement or more than one measurement over a period of time of the levels of at least a first marker and urotensin in a bodily fluid is made and compared to a standard model.

The invention will now be described further in the following non-limiting example. Reference is made to the accompanying drawings in which:
Figures 1a and 1b are graphs showing plasma NT proBNP and plasma UTN respectively according to NYHA class;

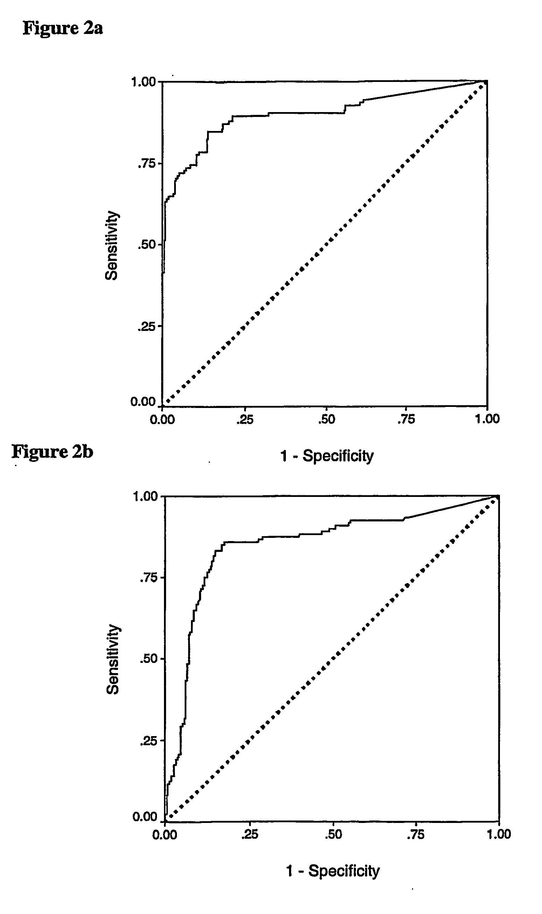

Figures 2a and 2b are receiver operating curves (ROC) for NT proBNP and UTN respectively in the diagnosis of heart failure; and

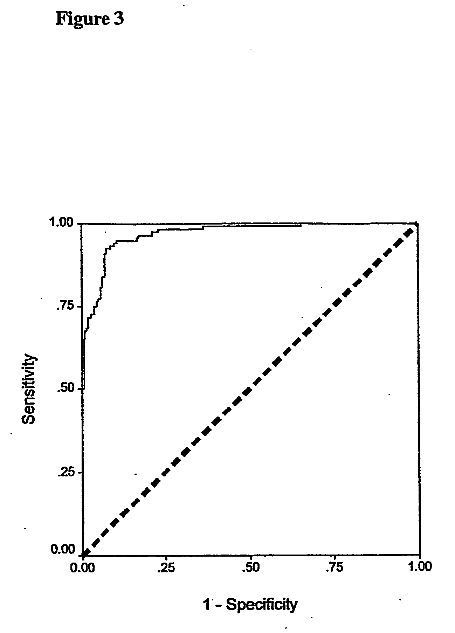

Figure 3 is a ROC curve prognostic index for combination of N-terminal proBNP and UTN in the diagnosis of heart failure.

### METHODS

### Plasma samples from normal controls and patients with heart failure.

Patients with heart failure were recruited from the Leicester Royal Infirmary clinics and wards. All had a clinical diagnosis of heart failure and echocardiographically confirmed ejection fractions below 45%. Normal controls were age and gender matched with these patients, were on no medication and had echocardiographically confirmed ejection fractions greater than 55%. Patient characteristics are reported in Table 1.

10 mls of blood was obtained by venepuncture after 15 min bed rest and mixed in ice-cold tubes containing EDTA and aprotinin. Plasma recovered following centrifugation was stored at -70°C until assayed.

### Assay of N-terminal proBNP

The assay for N-terminal proBNP was based on the non-competitive N-terminal proBNP assay described by Karl (Karl et al. Scand J Clin Lab Invest Suppl 1999;230:177-181). Rabbit polyclonal antibodies were raised to the N-terminal (amino acids 1-12) and C-terminal (amino acids 65-76) of the human N-terminal proBNP.

IgG from the sera was purified on protein A sepharose columns. The C-terminal directed antibody (0.5 µg in 100 µl for each ELISA plate well) served as the capture antibody. The N-terminal antibody was affinity purified and biotinylated. Aliquots (20µL) of samples or N-BNP standards were incubated in the C-terminal antibody coated wells with the biotinylated antibody for 24 hours at 4°C. Following washes, streptavidin labeled with methyl-acridinium ester (streptavidin-MAE, 5 x 10⁶ relative light units /ml) (Hart & Taaffe, J Immunol Methods 1987;101: 91-96) was added to each well. Plates were read on a Dynatech MLX Luminometer as previously described (Hughes et al. Clin Sci 1999; 96 : 373-380). The lower limit of detection was 5.7 fmol/ml of unextracted plasma. Within and between assay coefficients of variation were acceptable at 2.3% and 4.8% respectively. There was no cross-reactivity with ANP, BNP or CNP.

### Assay of Urotensin II

Antibody specific for the cyclic form of UTN was obtained from Phoenix Pharmaceuticals Inc., Belmont, CA. Biotinylated UTN purified on reverse phase HPLC served as the tracer. A competitive assay using C₁₈ extracts of plasma was utilized, incubating 50 ng of the antibody with extracts or standards (ranging from 1 to 2000 fmol per well) in 100 µl of assay buffer (as described in 12). After 24 h of incubation at 4°C, the biotinylated UTN tracer was added (250 fmols per well). Immunoprecipitates were recovered in ELISA plates coated with anti-rabbit IgG (100 ng/well). Following washes and incubation with streptavidin-MAE, chemiluminescence was elicited as described above. Intra- and interassay coefficients of variation were 2.3 and 8.1 % respectively, with no reactivity for BNP or N-terminal proBNP. The lower limit of detection was 3.1 fmol/ml.

### Statistical Analysis

Statistical analyses were performed on SPSS Version 11. Data are presented as medians [ranges]. Comparisons were by Kruskal-Wallis analysis of variance and receiver operating characteristic (ROC) curves were plotted. Correlation analysis employed Spearman's rho (rₛ). A value of P<0.05 was considered statistically significant.

### RESULTS

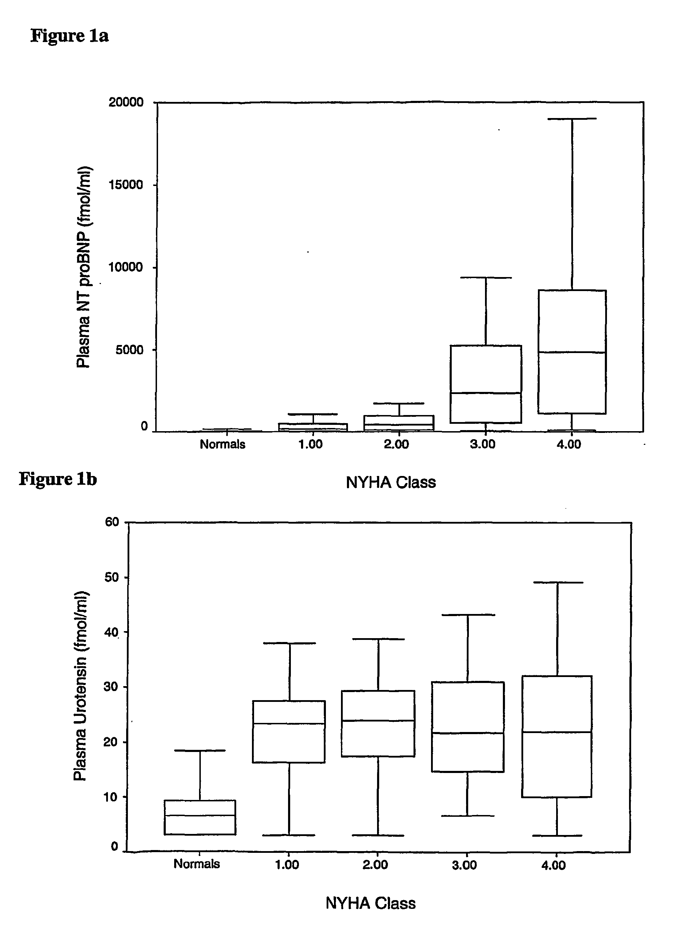

Table 1 illustrates the characteristics of the normal and heart failure patients, who were well matched for age and gender. As expected, N-terminal proBNP was significantly elevated in heart failure patients. In the normal population, there was a positive correlation of N-terminal proBNP with increasing age (rₛ = 0.41, P<0.001) and females had higher levels than males (P<0.001, Table 1). N-terminal proBNP increased with increasing NYHA class (Figure 1a, P<0.001 by Kruskal Wallis test). Plasma UTN was also elevated in heart failure patients (Table 1), but there was no correlation with age. In contrast to N-terminal proBNP, levels were lower in females compared to males (P<0.001, Table 1). Plasma UTN was not affected by increasing NYHA class (Figure 1b). Both N-terminal proBNP and UTN were elevated in heart failure patients irrespective of gender (P<0.001 for all comparisons). N-terminal proBNP and UTN were also modestly correlated (rₛ = 0.35, P<0.001).

In the heart failure patients, plasma UTN levels were not dependent on use of diuretics, beta blockers or ACE inhibitors. ROC curves for the detection of heart failure for both peptides revealed areas of 0.90 and 0.86 for N-terminal proBNP and UTN respectively (P<0.001 compared to the diagonal reference line).

Using the univariate general linear model procedure on SPSS, and entering age as a covariate and gender and NYHA class as factors, analysis of the log normalised N-terminal proBNP levels in the heart failure patients yielded an r² of 0.446 for the model (P<0.001) with age, gender and NYHA class as significant predictive variables (P<0.034, 0.002 and 0.001 respectively). None of these factors was identified as predictive variables of UTN (r² = 0.058). Thus, UTN levels in heart failure patients are elevated irrespective of age, gender or NYHA class.

From the comparison of the graphs in Figures 1a and 1b, one may see that the measurement of plasma UTN in combination with that of plasma NT proBNP is able to yield greater information concerning the diagnosis of heart failure than measurement of NT proBNP alone. Namely, levels of NT ProBNP only start to become elevated upon progression to NYHA Class 3, whereas plasma UTN levels are shown to be elevated in patients with an NYHA class of greater than 1. Thus, measurement of these two markers can lead to early stage identification of heart disease.

**Table 1. Patient characteristics. Medians [ranges] are reported and P values were computed using the Kruskal-Wallis or Mann Whitney tests (comparing normal and heart failure patients).**

| | **Normal controls** | **Heart failure patients** | **P value** |
|---|---|---|---|
| **Number** | 220 (78 (35%) female) | 126 (37 (29%) female) | ns for gender |
| **Age (years)** | 61.3 [26-80.6] | 63 [20-87] | ns |
| **Drug therapy** | None | | |
| Diuretics | | 98 | |
| β blockers | | 47 | |
| ACE inhibitors | | 99 | |
| **Aetiology** | | | |
| Ischaemic cardiomyopathy | | 83 | |
| Dilated cardiomyopathy | | 32 | |
| Hypertensive cardiomyopathy | | 7 | |
| Valvular disease | | 4 | |
| **NT proBNP levels** | | | |
| All | 21.4 [5.7-991.9] | 657 [6-29368] | 0.001 |
| Male | 12.5 [5.7-631.2] | 464 [6-25182] | 0.001 |
| Female | 47.7 [5.7-991.9] | 3127 [104-29368] | 0.001 |
| **UTN levels** | | | |
| All | 6.6 [3.1-42.6] | 22.1 [3.1-49.2] | 0.001 |
| Male | 7.2 [3.1-42.6] | 22.4 [3.1-46.7] | 0.001 |
| Female | 4.6 [3.1-17.4] | 20.6 [3.1-49.2] | 0.001 |

Although this study considered the levels of UTN and NT-proBNP in plasma, one would also consider analysis of other sources of bodily fluid, namely, whole blood, serum, interstitial fluid or urine. Furthermore, one would expect that BNP instead of NT-proBNP could be monitored in conjunction with plasma UTN to yield similar information concerning diagnosis of heart disease since BNP is also expressed along with NT-proBNP in plasma.

### DISCUSSION

This study demonstrates for the first time that plasma UTN levels are very significantly elevated in patients with heart failure, in conjunction with the raised N-terminal proBNP levels previously described (Hunt et al. Clin Endocrinol 1997; 47 : 287-296; Hughes et al. Clin Sci 1999; 96 : 373-380). Thus, the increased UTN expression in human myocardium from patients with heart failure (Douglas et al. Lancet 2002; 359 : 1990-1997) is reflected also in plasma. Although plasma N-terminal proBNP is elevated with age and female gender, such age trends are not evident for UTN in the normal population. These age and gender differences in peptide measurements emphasise the importance of matching patients and normal controls for both age and gender as was achieved in this study. The correlation of the two plasma peptides is very much in keeping with the *in vitro* observation that UTN increases myocardial BNP expression (Zou et al. FEBS Lett 2001; 508 : 57-60). In contrast to N-terminal proBNP, where levels rise with NYHA class, UTN levels were elevated in NYHA class I patients, with no further change for more severe disease. Whether this reflects differences in clearance rates with disease severity remains to be investigated. For the detection of heart failure, the area under the ROC curve for UTN is comparable to that of N-terminal proBNP, indicating a use for the peptide in accurate diagnosis of heart failure. A particularly useful feature of UTN for diagnosis of heart failure is its independence of factors such as age, gender and NYHA class in the patients. N-terminal proBNP in contrast is very significantly affected by all these variables.

The source of the UTN in plasma is currently unknown, but may be cardiovascular in origin, due to the documented increased expression in cardiomyocytes, endothelium, vascular myocytes and inflammatory cells (Douglas et al. Lancet 2002; 359 : 1990-1997). Taken in conjunction with this evidence, our data suggests that the UTN system is likely to be involved in the pathophysiology of heart failure. It is currently unclear whether the increased activity is causal to or an effect of ventricular dysfunction.

The involvement of this new cardiovascular peptide in heart failure pathophysiology suggests an additional hormonal system that could in the future be modulated with appropriate receptor agonists or antagonists. Its use as a diagnostic test for heart failure may be more immediate.

## Claims

1. A method for the diagnosis of heart failure in a mammalian subject whereby the level of urotensin in a sample of bodily fluid is measured and compared to reference levels typically found in the said bodily fluid of a subject which are indicative of the absence of heart failure, whereby an elevated level of urotensin is indicative of heart failure.

2. A method according to claim 1, whereby the level of urotensin is determined by use of an immunoassay.

3. A method according to claim 1 or claim 2, wherein the level of at least a further marker indicative of heart failure is additionally measured and compared to reference levels typically found in the said bodily fluid of a mammalian subject which are indicative of the absence of heart failure, whereby an elevated level of the at least a further marker is indicative of heart failure.

4. A method according to claim 3, wherein the further marker is NT-proBNP or BNP.

5. A method according to claim 3 or claim 4, whereby the level of further marker is determined by use of an immunoassay.

6. A method according to any preceding claim, wherein the bodily fluid is plasma or interstitial fluid.

7. A method according to any preceding claim, wherein the subject is human.

8. A method of tracking and staging of the degree of severity of heart failure in a human subject whereby one measurement or more than one measurement over a period of time of the levels of urotensin in a sample of bodily fluid and at least a further marker indicative of heart failure is made and compared to a standard model.

9. A method according to claim 8, wherein the standard model is a standard model of levels of analyte versus disease status.

10. A method according to claim 8 or claim 9, whereby the staging is determined according to the new York heart association (NYHA) classification.

## Patentansprüche

1. Verfahren zur Diagnose von Herzinsuffizienz bei einem Individuum, das ein Säugetier ist, wobei der Urotensin-Level in einer Probe Körperflüssigkeit gemessen und mit Referenzleveln verglichen wird, die in dieser Körperflüssigkeit eines Individuums für gewöhnlich gefunden werden und für ein Fehlen einer Herzinsuffizienz indikativ sind, wobei ein erhöhter Urotensin-Level für Herzinsuffizienz indikativ ist.

2. Verfahren nach Anspruch 1, wobei der Urotensin-Level durch Verwendung eines Immunassays bestimmt wird.

3. Verfahren nach Anspruch 1 oder 2, wobei der Level zumindest eines weiteren Markers, der für Herzinsuffizienz indikativ ist, zusätzlich gemessen und mit Referenzleveln verglichen wird, die in dieser Körperflüssigkeit eines Individuums, das ein Säugetier ist, für gewöhnlich gefunden werden und für das Fehlen einer Herzinsuffizienz indikativ sind, wobei ein erhöhter Level des zumindest einen weiteren Markers für Herzinsuffizienz indikativ ist.

4. Verfahren nach Anspruch 3, wobei der weitere Marker NT-proBNP oder BNP ist.

5. Verfahren nach Anspruch 3 oder 4, wobei der Level des weiteren Markers durch Verwendung eines Immunassays bestimmt wird.

6. Verfahren nach einem vorherigen Anspruch, wobei die Körperflüssigkeit Plasma oder interstitielle Flüssigkeit ist.

7. Verfahren nach einem vorherigen Anspruch, wobei das Individuum ein Mensch ist.

8. Verfahren zum Aufspüren ("tracking") von Herzinsuffizienz und zum Einteilen des Schweregrades der Herzinsuffizienz in Stadien ("staging") bei einem menschlichen Individuum, wobei eine Messung oder mehrere Messungen der Urotensin-Level in einer Probe Körperflüssigkeit und der Level eines weiteren Markers, der für Herzinsuffizienz indikativ ist, über einen Zeitraum durchgeführt und mit einem Standardmodell verglichen werden.

9. Verfahren nach Anspruch 8, wobei das Standardmodell ein Standardmodell von Analyt-Leveln gegen Krankheitszustand ist.

10. Verfahren nach Anspruch 8 oder 9, wobei das Staging gemäß der Klassifizierung der "New York Heart Association" (NYHA) erfolgt.

## Revendications

1. Procédé pour le diagnostic d'une attaque cardiaque chez un sujet mammalien par lequel le niveau d'urotensine dans un échantillon de fluide corporel est mesuré et comparé à des niveaux de référence typiquement trouvés dans ledit fluide corporel d'un sujet, lesquels sont indicatifs de l'absence d'attaque cardiaque, par lequel un niveau élevé d'urotensine est indicatif d'une attaque cardiaque.

2. Procédé selon la revendication 1, par lequel le niveau d'urotensine est déterminé par l'utilisation d'un dosage immunologique.

3. Procédé selon la revendication 1 ou la revendication 2, dans lequel le niveau d'au moins un marqueur supplémentaire indicatif d'une attaque cardiaque est en outre mesuré et comparé à des niveaux de référence typiquement trouvés dans ledit fluide corporel d'un sujet mammalien, lesquels sont indicatifs de l'absence d'attaque cardiaque, par lequel un niveau élevé d'au moins un marqueur supplémentaire est indicatif d'une attaque cardiaque.

4. Procédé selon la revendication 3, dans lequel le marqueur supplémentaire est NT-proBNP ou BNP.

5. Procédé selon la revendication 3 ou la revendication 4, par lequel le niveau du marqueur supplémentaire est déterminé par l'utilisation d'un dosage immunologique.

6. Procédé selon l'une quelconque des revendications précédentes, dans lequel le fluide corporel est du plasma ou du liquide interstitiel.

7. Procédé selon l'une quelconque des revendications précédentes, dans lequel le sujet est un humain.

8. Procédé de suivi et de classement du degré de gravité d'une attaque cardiaque chez un sujet humain par lequel une mesure ou plus d'une mesure sur une période de temps des niveaux d'urotensine dans un échantillon de fluide corporel et au moins un marqueur supplémentaire indicatif d'une attaque cardiaque sont faites et comparées à un modèle normalisé.

9. Procédé selon la revendication 8, dans lequel le modèle normalisé est un modèle normalisé de niveaux d'analyte en fonction de l'état de la maladie.

10. Procédé selon la revendication 8 ou la revendication 9, par lequel le classement est déterminé selon la classification de la New York Heart Association (NYHA).
